(19)

Europäisches Patentamt
European Patent Office
Office européen des brevets

(11) **EP 3 333 162 A1**

(12) **EUROPEAN PATENT APPLICATION**

(43) Date of publication:
**13.06.2018 Bulletin 2018/24**

(51) Int Cl.:
*C07D 401/04* (2006.01)

(21) Application number: **16460092.6**

(22) Date of filing: **12.12.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Silesian Catalysts sp. z o.o.**
**54-066 Wroclaw (PL)**

(72) Inventors:
- **NIKLEWICZ, Pawel Grzegorz**
  **50-570 Wroclaw (PL)**
- **LAPCZYNSKI, Radoslaw Mieczyslaw**
  **58-160 Swiebodzice (PL)**
- **PLUTA, Janusz**
  **54-245 Wroclaw (PL)**
- **OWCZAREK, Artur**
  **50-148 Wroclaw (PL)**
- **KAROLEWICZ, Bozena**
  **51-124 Wroclaw (PL)**

(54) **METOD FOR PREPARING N-(2-METHYL-5-NITROPHENYL)-4-(PYRIDIN-3-YL)PYRIMIDIN-2-AMINE**

(57) The invention relates to a method for preparing N-(2-Methyl-5-nitrophenyl)-4-(pyridin-3-yl)pyrimidin-2-amine, a compound from the group of 2-aminopyrimidine derivatives, which is the precursor of active pharmaceutical ingredients, in particular imatinib.

The method of production of N-(2-methyl-5-nitrophenyl)-4-(pyridin-3-yl)pyrimidin-2-amine or other 2-pyrimidineamine lies in that 3-(dimethylamino)-1-(3-pyridyl)prop-2-en-1-one, after being mixed with 1-(2-methyl-5-nitrophenyl)-guanidine nitrate and an alkali hydroxide, preferably sodium hydroxide dissolved in a solvent in the form of dimethyl sulfoxide (DMSO), is subjected to coupling reaction under a pressure from 1 to 50 MPa in a rotating microwave field, with an absorbed microwave power volumetric density contained in the range from 1 $W/cm^3$ to 1 $MW/cm^3$. The coupling process is preferably conducted at a temperature from 300 to 773K, wherein the volumetric density of microwave power absorbed by the reaction system under isothermal process conditions is controlled during the synthesis process.

In a variant of the method, the reagents are subjected to coupling reaction in a microwave reactor, in which the reaction vessel is placed in a container equipped with at least two microwave ports supplying microwave energy to the reagents positioned in the reaction vessel by means of electrodes that are isolated from the reagents by a dielectric layer and play the role of coupling elements.

Formula 1

**Description**

The subject of the invention is the method of preparing N-(2-methyl-5-nitrophenyl)-4-(pyridin-3-yl)pyrimidin-2-amine, a compound from the group of 2-aminopyrimidine derivatives, which is the precursor of Active Pharmaceutical Ingredients (APIs).

The derivative of N-(2-Methyl-5-nitrophenyl)-4-(pyridin-3-yl)pyrimidin-2-amine is a substrate for production of imatinib mesylate and 4-methyl-3-(4-pyridin-3-yl-pyrimidin-2-yl-amine)-phenylamine-2,5-dichloro-4-hydroxy-3,6-dioxo-cyclohexa-1,4-dienol (chloranilic acid N-(5-nitro-2-methylphenyl)-4-(3-pyridyl)-2-pyrimidineamine salt), a substance with antibacterial and antifungal activity as confirmed by *in vitro* tests.

Introduced to medical practice in 2001, imatinib mesylate, as a tyrosine kinase inhibitor, is used for production of antineoplastic drugs of high effectiveness and low general toxicity. Imatinib mesylate is used primarily in the treatment of chronic myelogenous leukemia with the presence of the Philadelphia chromosome and acute lymphoblastic leukemia, and in the treatment of mesenchymal neoplasms of alimentary tract cells and skin fibrosarcomata.

For the synthesis of imatinib, various substrates can be used, the choice of which and whether they already contain a heterocyclic ring with two nitrogen atoms or not determines subsequent operations, such as possible substitution of a nitro group or a lower aldoxyl group for the benzene ring carbon or the substitution of halogens for the carbon atom in the ring. According to patent EP 0 564 409, N-(2-methyl-5-nitrophenyl)-4-(pyridin-3-yl)pyrimidin-2-amine is used for the synthesis of imatinib. The imatinib production method known from international application WO03066613 uses N-(3-bromo-4-methylphenyl)-4-((4-methylpiperazin-1-yl)methyl)benzamide, which is condensed with 4-(pyridin-3-yl)pyrimidin-2-yl)-amine. The synthesis disclosed in international application WO2004/108699 uses 4-chlorophenyl)-N-{4-methyl-3-[4-(pyridin-3-yl)-pyrimidin-2-yl-amino]phenyl}-benzamide, which is condensed with N-methylpiperazine.

Another tyrosine kinase inhibitor in the form of 4-metylo-N-[3-(4-methyl-1H-imidazol-1-yl)-5-(trifluoromethyl)phenyl]-3-[4-pyridin-3-yl-pyrimid-in-2-yl)amino]benzamide, obtained from N-(2-Methyl-5-nitrophenyl)-4-(pyridin-3-yl)pyrimidin-2-amine, constitutes an active pharmaceutical ingredient known as nilotinib. This compound, similarly as imatinib, selectively inhibits proliferation and induces apoptosis in the cell lines and primary cells of Philadelphia chromosome leukemia in patients with chronic lymphoblastic leukemia.

The methods of obtaining N-(5-Nitro-2-methylphenyl)-4-(3-pyridyl)-2-pyrimidineamine known from prior art involve many stages, are time-consuming and require the purification of intermediate products at successive stages of synthesis, which further reduces the overall product yield. The problem needed to be solved in production of the derivatives of 2-aminopyridines, including N-(2-methyl-5-nitrophenyl)-4-(pyridin-3-yl)pyrimidin-2-amine, necessary for the production of active pharmaceutical ingredients, is to develop a method that would increase the yield of the substance synthesis process and enhance the product purity profile. It is also important to remove, often harmful, residues of organic solvents and to eliminate the incomplete homogeneity of the obtained precursors of medicinal substances, despite the high production costs resulting from the multi-stage and time-consuming processes.

Patent EP 1833815 discloses the method of producing the 2-aminopyridine derivative, specifically N-(5-Nitro-2-methylphenyl)-4-(3-pyridyl)-2-pyrimidineamine, using the tank reactor. The process consists in many-hour heating of a mixture of 3-(dimethylamino)-1-(3-pyridyl)prop-2-en-1-one and 1-(2-methyl-5-nitrophenyl)guanidine nitrate dissolved in dimethylformamide (DMF) in the presence of sodium hydroxide.

The Patent Specification EP 0 564 409 teaches the synthesis of imatinib, whereby, at the first stage, 2-methyl-5-nitroaniline is transformed into 2-methyl-5-nitrophenyl guanidine nitrate which, at the second stage, is condensed with 3-(dimethylamino)-1-(pyridin-3-yl)prop-2-en-1-one to obtain N-(2-Methyl-5-nitrophenyl)-4-(pyridin-3-yl)pyrimidin-2-amine. As a result of the catalytic reduction of the nitro group to the amine group in the latter compound, N-(2-Methyl-5-nitrophenyl)-4-(pyridin-3-yl)pyrimidin-2-amine is obtained, which, in turn, is acylated with 4-(4-methylpiperazin-1-yl-methyl)benzoyl chloride.

The patent and scientific literature has not reported so far an efficient method for conducting the processes of production of N-(2-methyl-5-nitrophenyl)-4-(pyridin-3-yl)pyrimidin-2-amine or other 2-pyrimidineamine derivatives under high pressure conditions with the participation of microwaves.

The essence of the method of production of N-(2-methyl-5-nitrophenyl)-4-(pyridin-3-yl)pyrimidin-2-amine or other 2-pyrimidineamine, as represented with formula 1, lies in that 3-(dimethylamino)-1-(3-pyridyl)prop-2-en-1-one, after being mixed with 1-(2-methyl-5-nitrophenyl)guanidine nitrate and an alkali hydroxide, preferably sodium hydroxide dissolved in a solvent in the form of dimethyl sulfoxide (DMSO), is subjected to coupling reaction under a pressure from 1 to 50 MPa, preferably 25 MPa, in a rotary microwave field, with an absorbed microwave power volumetric density contained in the range from 1 W/cm$^3$ to 1 MW/cm$^3$. The coupling process is preferably conducted at a temperature from 300 do 773K, most preferably at 463K.

Furthermore, the volumetric density of microwave power absorbed by the reaction system under isothermal process conditions is preferably controlled during the synthesis process according to the equation:

$$G = \frac{P}{V}$$

where:

G - denotes the volumetric density of absorbed power [W/cm³],
P - absorbed power [W],
V - volume of the reactor's reaction space [cm³].

The volumetric density of the absorbed microwave power is preferably contained in the range from 20 W/cm³ to 500 W/cm³, and most preferably it is 52.35 W/cm³.

In a variant of the method, the reagents are subjected to coupling reaction in a microwave reactor, in which the reaction vessel is placed in a container equipped with at least two microwave ports supplying microwave energy to the reagents positioned in the reaction vessel by means of electrodes that are isolated from the reagents by a dielectric layer and play the role of coupling elements. The remaining electrodes are distributed uniformly along the circle and are supplied from a microwave power source with a phase shift by an angle of 360°/N, where N is the number of electrodes. The reactor allows the method according to the invention to be carried out in the flow of the reagents by a continuous method. Whereas, the high yield reagent reactor residence time is used in the range from 1 millisecond to 30 minutes, preferably 10 minutes.

The method of production of N-(2-methyl-5-nitrophenyl)-4-(pyridin-3-yl)pyrimidin-2-amine according to the invention guarantees a fast and controlled synthesis process, whereas, it enables a high-purity product to be obtained precisely and selectively and at high quantitative yield. The application of the method considerably shortens the reagent synthesis time and enhances the purity of the obtained products with an increased energy efficiency of the process. The reduced demand for the reagents, the possibility of using cheaper solvents, the improvement in the quality of the obtained synthesis products, and the control of the synthesis process being much better compared to traditional methods, including also processes carried out in containers, are all not without significance, either.

An advantage of the invention is the fact that using microwaves as the source of energy for the reaction solutions improves the yield and reduces the time of the synthesis, while enhancing the purity of the synthesis products. This is important, as the overall output of conventional synthesis is often very low due to the length of the process and its multi-stage nature.

As against synthesis conducted in tank reactors, in the process carried out in the microwave reactor known from Polish Patent Application No. P.395 891 no reagent concentration gradients nor temperature gradients occur, and the duration of reaction mix heating up and cooling down to preset process parameters is reduced. This reactor provides a highly homogeneous distribution of the rotating microwave field with the precise control of the field shape, which allows measuring sensors to be introduced into the reactor. Using the flow reactor for the method according to the invention significantly enhances the technical security of chemical compound synthesis processes and allows them to be conducted in a continuous manner.

The subject of the invention is illustrated by examples of the embodiment of the method.

Example 1

A previously prepared reaction mix dissolved in dimethyl sulfoxide (DMSO), containing: 3-(dimethylamino)-1-(3-pyridyl)prop-2-en-1-one at a molar concentration of 2.19 mol/dm³, 1-(2-methyl-5-nitrophenyl)guanidine nitrate at a molar concentration of 2.19 mol/dm³ and sodium hydroxide at a molar concentration of 2.19 mol/dm³, was passed through the pressure microwave reactor specified in Polish Patent Application No. P.395 891. The reactor was equipped with four microwave antennas in a rotary system, which provided a homogeneous rotating microwave field. Microwave energy was supplied to individual microwave antennas with a wave phase shift of 90 degrees. The system was provided with a reflected power measurement capability. The high-pressure reaction chamber ensured isothermal conditions for running the process thanks to its construction featuring a thick steel jacket filled with Teflon. The temperature measurement was taken using a thermocouple positioned in the microwave field minimum within the reaction space. The control of synthesis process selectivity was achieved by controlling the parameter of the volumetric density of power absorbed by the reaction system under isothermal process conditions. The volumetric density of absorbed power was defined as the ratio of power absorbed by the system [W] to the volume of the reactor's reaction space [cm³], which, in the case at hand, was 5.73 cm³. The synthesis process was conducted with the following preset parameters: reaction chamber pressure, 25 MPa; flow rate, 0.573 ml/min; reagent reactor residence time, 10 min; chamber temperature, 463K; and absorbed power volumetric density, 52.35 W/cm³. The obtained reaction product, N-(2-methyl-5-nitrophenyl)-4-(pyridin-3-yl)pyrimidin-2-amine, was crystallized from the post-reaction mix, and then rinsed and dried. N-(2-methyl-5-nitrophenyl)-4-(pyridin-3-yl)pyrimidin-2-amine was obtained at an efficiency of 98.00 [w/w%] and a purity of 96.80%. The purity was confirmed by the $^1$H NMR, GC-MS and LC-MS techniques.

Example 2

A previously prepared reaction mix containing: 2.09 mol/dm$^3$ 3-(dimethylamino)-1-(3-pyridyl)prop-2-en-1-one, 2.09 mol/dm$^3$ 1-(2-methyl-5-nitrophenyl)guanidine nitrate and 2.09 mol/dm$^3$ sodium hydroxide dissolved in dimethyl sulfoxide (DMSO) was passed through the pressure microwave reactor. The reactor was equipped with four microwave antennas in a rotary system, which provided a homogeneous rotating microwave field. Microwave energy was supplied to individual microwave antennas with a wave phase shift of 80 degrees. The system was provided with a reflected power measurement capability. The high-pressure reaction chamber ensured isothermal conditions for running the process thanks to its construction featuring a thick steel jacket filled with Teflon. The temperature measurement was taken using a thermocouple positioned in the microwave field minimum within the reaction space. The control of synthesis process selectivity was achieved by controlling the parameter of the volumetric density of power absorbed by the reaction system under isothermal process conditions. The volumetric density of absorbed power was defined as the ratio of power absorbed by the system [W] to the volume of the reactor's reaction space [cm$^3$], which, in the case at hand, was 5.63 cm$^3$. The synthesis process was conducted with the following preset parameters: reaction chamber pressure, 25 MPa; flow rate, 0.563 ml/min; reagent reactor residence time, 10 min; chamber temperature, 453K; and absorbed power volumetric density, 52.35 W/cm$^3$.

The obtained reaction product, N-(2-methyl-5-nitrophenyl)-4-(pyridin-3-yl)pyrimidin-2-amine, was crystallized from the post-reaction mix, and then rinsed and dried. N-(2-methyl-5-nitrophenyl)-4-(pyridin-3-yl)pyrimidin-2-amine was obtained at an efficiency of 97.90 [w/w%] and a purity of 96.70%. The purity was confirmed by the $^1$H NMR, GC-MS and LC-MS techniques.

Example 3

A previously prepared reaction mix containing: 2.29 mol/dm$^3$ 3-(dimethylamino)-1-(3-pyridyl)prop-2-en-1-one, 2.29 mol/dm$^3$ 1-(2-methyl-5-nitrophenyl)guanidine nitrate and 2.29 mol/dm$^3$ sodium hydroxide dissolved in dimethyl sulfoxide (DMSO) was passed through the pressure microwave reactor. The reactor was equipped with four microwave antennas in a rotary system, which provided a homogeneous rotating microwave field. Microwave energy was supplied to individual microwave antennas with a wave phase shift of 90 degrees. The system was provided with a reflected power measurement capability. The high-pressure reaction chamber ensured isothermal conditions for running the process thanks to its construction featuring a thick steel jacket filled with Teflon. The temperature measurement was taken using a thermocouple positioned in the microwave field minimum within the reaction space. The control of synthesis process selectivity was achieved by controlling the parameter of the volumetric density of power absorbed by the reaction system under isothermal process conditions. The volumetric density of absorbed power was defined as the ratio of power absorbed by the system [W] to the volume of the reactor's reaction space [cm$^3$], which was 5.83 cm$^3$. The synthesis process was conducted with the following preset parameters: reaction chamber pressure, 25 MPa; flow rate, 0.583 ml/min; reagent reactor residence time, 10 min; chamber temperature, 473K; and absorbed power volumetric density, 52.35 W/cm$^3$. The obtained reaction product, N-(2-methyl-5-nitrophenyl)-4-(pyridin-3-yl)pyrimidin-2-amine, was crystallized from the post-reaction mix, and then rinsed and dried. N-(2-methyl-5-nitrophenyl)-4-(pyridin-3-yl)pyrimidin-2-amine was obtained at an efficiency of 97.00 [w/w%] and a purity of 95.80%. The purity was confirmed by the $^1$H NMR, GC-MS and LC-MS techniques.

Example 4

A previously prepared reaction mix containing: 2.09 mol/dm$^3$ 3-(dimethylamino)-1-(3-pyridyl)prop-2-en-1-one, 2.09 mol/dm$^3$ 1-(2-methyl-5-nitrophenyl)guanidine nitrate and 2.09 mol/dm$^3$ sodium hydroxide dissolved in dimethyl sulfoxide (DMSO) was passed through the pressure microwave reactor. The reactor was equipped with four microwave antennas in a rotary system, which provided a homogeneous rotating microwave field. Microwave energy was supplied to individual microwave antennas with a wave phase shift of 90 degrees. The system was provided with a reflected power measurement capability. The high-pressure reaction chamber ensured isothermal conditions for running the process thanks to its construction featuring a thick steel jacket filled with Teflon. The temperature measurement was taken using a thermocouple positioned in the microwave field minimum within the reaction space. The control of synthesis process selectivity was achieved by controlling the parameter of the volumetric density of power absorbed by the reaction system under isothermal process conditions. The volumetric density of absorbed power was defined as the ratio of power absorbed by the system [W] to the volume of the reactor's reaction space [cm$^3$], which, in the case at hand, was 5.73

cm$^3$. The synthesis process was conducted with the following preset parameters: reaction chamber pressure, 24 MPa; flow rate, 0.573 ml/min; reagent reactor residence time, 10 min; chamber temperature, 473K; and absorbed power volumetric density, 52.35 W/cm$^3$. The obtained reaction product, N-(2-methyl-5-nitrophenyl)-4-(pyridin-3-yl)pyrimidin-2-amine, was crystallized from the post-reaction mix, and then rinsed and dried. N-(2-methyl-5-nitrophenyl)-4-(pyridin-3-yl)pyrimidin-2-amine was obtained at an efficiency of 99.10 [w/w%] and a purity of 97.50%. The purity was confirmed by the $^1$H NMR, GC-MS and LC-MS techniques.

Example 5

A previously prepared reaction mix dissolved in dimethyl sulfoxide (DMSO), containing: 2.39 mol/dm$^3$ 3-(dimethylamino)-1-(3-pyridyl)prop-2-en-1-one, 2.39 mol/dm$^3$ 1-(2-methyl-5-nitrophenyl)guanidine nitrate and 2.39 mol/dm$^3$ sodium hydroxide dissolved in dimethyl sulfoxide (DMSO) was passed through the pressure microwave reactor. The reactor was equipped with four microwave antennas in a rotary system, which provided a homogeneous rotating microwave field. Microwave energy was supplied to individual microwave antennas with a wave phase shift of 90 degrees. The system was provided with a reflected power measurement capability. The high-pressure reaction chamber ensured isothermal conditions for running the process thanks to its construction featuring a thick steel jacket filled with Teflon. The temperature measurement was taken using a thermocouple positioned in the microwave field minimum within the reaction space. The control of synthesis process selectivity was achieved by controlling the parameter of the volumetric density of power absorbed by the reaction system under isothermal process conditions. The volumetric density of absorbed power was defined as the ratio of power absorbed by the system [W] to the volume of the reactor's reaction space [cm$^3$], which, in the case at hand, was 5.83 cm$^3$. The synthesis process was conducted with the following preset parameters: reaction chamber pressure, 25 MPa; flow rate, 0.583 ml/min; reagent reactor residence time, 10 min; chamber temperature, 473K; and absorbed power volumetric density, 53.35 W/cm$^3$. The obtained reaction product, N-(2-methyl-5-nitrophenyl)-4-(pyridin-3-yl)pyrimidin-2-amine, was crystallized from the post-reaction mix, and then rinsed and dried in the well-known manner. N-(2-methyl-5-nitrophenyl)-4-(pyridin-3-yl)pyrimidin-2-amine was obtained at an efficiency of 98.80 [w/w%] and a purity of 96.95%. The purity was confirmed by the 1H NMR, GC-MS and LC-MS techniques.

The efficacy and effectiveness of the method of production of N-(2-methyl-5-nitrophenyl)-4-(pyridin-3-yl)pyrimidin-2-amine according to the invention was confirmed by tests carried out for four different mole ratios of the reagents: 3-(dimethylamino)-1-(3-pyridyl)prop-2-en-1-one (Reagent 1) and 1-(2-methyl-5-nitrophenyl)guanidine nitrate (Reagent 2) defined in Table 1 in the reaction mix, subjected to coupling reaction in the microwave tank reactor and to coupling reaction in the high-pressure flow microwave reactor with a rotating magnetic field.

Table 1. Mole ratios of the substrates for four examined variants.

| Sample number | Reagent 1 [mmol] | Reagent 2 [mmol] | NaOH [mmol] | DMSO [ml] |
|---|---|---|---|---|
| 1 | 54.76 | 54.75 | 55.00 | 25 |
| 2 | 54.74 | 54.75 | 55.00 | 25 |
| 3 | 27.38 | 27.38 | 55.00 | 25 |
| 4 | 27.38 | 27.38 | 27.50 | 25 |

Table 2. Comparison of the yield and purity of the N-(2-methyl-5-nitrophenyl)-4-(pyridin-3-yl)pyrimidin-2-amine product obtained in the high-pressure rotating field flow microwave reactor, with reagent mole ratios as given in Table 2, with the process conducted in the microwave tank reactor.

| Coupling parameters | Time [min] | Power [W] | Reflection [W] | T [°C] | Yield [w/w%] | Purity [%] |
|---|---|---|---|---|---|---|
| tank microwave | 10 | 150 | ~60 | 185-190 | 62.87 | 46.55 |
| rotating field flow microwave | 10 | 225 | 10 | 190 | 98.00 | 96.80 |

As a result, in the case of conducting the coupling reaction of 3-(dimethylamino)-1-(3-pyridyl)prop-2-en-1-one (Reagent 1) and 1-(2-methyl-5-nitrophenyl)guanidine nitrate (Reagent 2) in the high-pressure rotating field flow microwave reactor, with the identical reagent mole rations, a significant increase in the yield and an over twofold increase in the purity of the N-(2-methyl-5-nitrophenyl)-4-(pyridin-3-yl)pyrimidin-2-amine product was achieved, compared to the result of coupling reaction conducted in the tank microwave reactor.

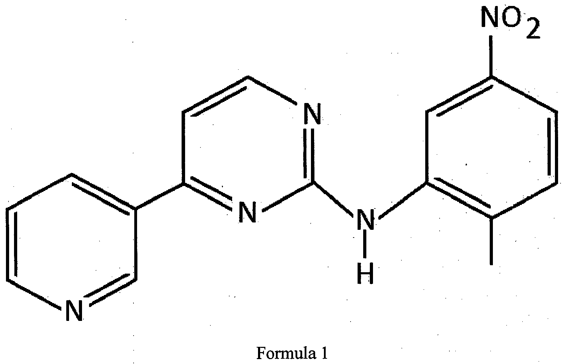

**Claims**

1. The method for preparing N-(2-methyl-5-nitrophenyl)-4-(pyridin-3-yl)pyrimidin-2-amine, represented with formula 1, **characterized in that** 3-(dimethylamino)-1-(3-pyridyl)prop-2-en-1-one after being mixed with 1-(2-methyl-5-nitrophenyl)-guanidine nitrate and an alkali hydroxide, preferably sodium hydroxide dissolved in a solvent in the form of dimethyl sulfoxide (DMSO), is subjected to coupling reaction under a pressure from 1 to 50 MPa in a rotating microwave field, with an absorbed microwave power volumetric density contained in the range from 1 W/cm$^3$ to 1 MW/cm$^3$.

2. The method according to claim 1, **characterized in that** the coupling of the mix of the substrates is conducted at a temperature from 300 to 773K and under a pressure of 25 MPa.

3. The method according to claim 2, **characterized in that** the coupling of the mix of the substrates is conducted at a temperature of 463K.

4. The method according to claim 1, **characterized in that** the volumetric density of microwave power absorbed by the reaction system under isothermal process conditions according to the following equation is controlled during the synthesis process:

$$G = \frac{P}{V}$$

where:

   $G$ - denotes the volumetric density of absorbed power [W/cm$^3$],
   $P$ - absorbed power [W],
   $V$ - volume of the reactor's reaction space [cm$^3$].

5. The method according to claim 1, **characterized in that** the volumetric density of absorbed microwave power is contained in the range from 20 W/cm$^3$ to 500 W/cm$^3$.

6. The method according to claim 5, **characterized in that** the volumetric density of absorbed microwave power is 52.35 W/cm$^3$.

7. The method according to claim 1, **characterized in that** the reagents are subjected to coupling reaction in a microwave reactor, in which the reaction vessel is positioned in a container equipped with at least two microwave ports supplying microwave energy to the reagents placed in the reaction vessel through the agency of electrodes that are isolated from the reagents by a dielectric layer and perform the function of microwave coupling elements, and in which, furthermore, the remaining electrodes are uniformly distributed along the circle and supplied from a microwave power source with a phase shift by an angle of 360°/N, where N is the number of electrodes.

8. The method according to claim 7, **characterized in that** the coupling process is conducted in the flow of reagents by a continuous method.

9. The method according to claim 7, **characterized in that** the time of reagent residence in the reactor is used in the range from 1 millisecond to 30 minutes.

Formula 1

Europäisches
Patentamt

European
Patent Office

Office européen
des brevets

## EUROPEAN SEARCH REPORT

Application Number

EP 16 46 0092

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | EP 0 564 409 A1 (CIBA GEIGY AG [CH]) 6 October 1993 (1993-10-06) * example 1 * | 1-9 | INV. C07D401/04 |
| A,D | WO 2006/071130 A2 (INST FARMACEUTYCZNY [PL]; SZCZEPEK WOJCIECH [PL]; LUNIEWSKI WOJCIECH []) 6 July 2006 (2006-07-06) * example 2 * | 1-9 | |
| A | WO 2008/137794 A1 (IRM LLC; NOVARTIS AG [CH]; LI XIAOLIN [US]; LIU XIAODONG [US]; MOLTENI) 13 November 2008 (2008-11-13) * page 32, synthesis of compound 21 * | 1-9 | |

TECHNICAL FIELDS
SEARCHED        (IPC)

C07D

The present search report has been drawn up for all claims

| Place of search | Date of completion of the search | Examiner |
|---|---|---|
| Munich | 10 March 2017 | Bakboord, Joan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

....................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 16 46 0092

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2017

| Patent document cited in search report | | Publication date | Patent family member(s) | | Publication date |
|---|---|---|---|---|---|
| EP 0564409 | A1 | 06-10-1993 | AT | 188964 T | 15-02-2000 |
| | | | AU | 3569493 A | 07-10-1993 |
| | | | BR | 1100739 A | 06-06-2000 |
| | | | CA | 2093203 A1 | 04-10-1993 |
| | | | CN | 1077713 A | 27-10-1993 |
| | | | CY | 2229 B1 | 18-04-2003 |
| | | | CZ | 9300560 A3 | 16-02-1994 |
| | | | DE | 10299016 I1 | 29-08-2002 |
| | | | DE | 10299016 I2 | 24-08-2006 |
| | | | DE | 59309931 D1 | 24-02-2000 |
| | | | DK | 0564409 T3 | 19-06-2000 |
| | | | EP | 0564409 A1 | 06-10-1993 |
| | | | ES | 2142857 T3 | 01-05-2000 |
| | | | FI | 931458 A | 04-10-1993 |
| | | | GR | 3032927 T3 | 31-07-2000 |
| | | | HU | 226488 B1 | 02-03-2009 |
| | | | HU | 227080 B1 | 28-06-2010 |
| | | | IL | 105264 A | 11-04-1999 |
| | | | JP | 2706682 B2 | 28-01-1998 |
| | | | JP | H0687834 A | 29-03-1994 |
| | | | LU | 90908 I2 | 30-04-2003 |
| | | | NL | 300086 I1 | 01-05-2002 |
| | | | NO | 931283 A | 04-10-1993 |
| | | | NZ | 247299 A | 26-07-1995 |
| | | | PT | 564409 E | 30-06-2000 |
| | | | RU | 2125992 C1 | 10-02-1999 |
| | | | SG | 43859 A1 | 14-11-1997 |
| | | | SK | 28093 A3 | 06-04-1994 |
| | | | TW | 225528 B | 21-06-1994 |
| | | | ZA | 9302397 B | 04-10-1993 |
| WO 2006071130 | A2 | 06-07-2006 | AT | 481398 T | 15-10-2010 |
| | | | EP | 1833815 A2 | 19-09-2007 |
| | | | SI | 1833815 T1 | 31-01-2011 |
| | | | US | 2008194819 A1 | 14-08-2008 |
| | | | WO | 2006071130 A2 | 06-07-2006 |
| WO 2008137794 | A1 | 13-11-2008 | AU | 2008247442 A1 | 13-11-2008 |
| | | | BR | PI0811516 A2 | 18-11-2014 |
| | | | CA | 2686382 A1 | 13-11-2008 |
| | | | CN | 101720322 A | 02-06-2010 |
| | | | EA | 200901486 A1 | 30-06-2010 |
| | | | EP | 2152688 A1 | 17-02-2010 |
| | | | JP | 5160637 B2 | 13-03-2013 |
| | | | JP | 2010526151 A | 29-07-2010 |
| | | | KR | 20090130427 A | 23-12-2009 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 16 46 0092

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2017

| Patent document cited in search report | Publication date | Patent family member(s) | Publication date |
|---|---|---|---|
| | | US 2010234406 A1 | 16-09-2010 |
| | | WO 2008137794 A1 | 13-11-2008 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0564409 A
- WO 03066613 A
- WO 2004108699 A
- EP 1833815 A
- PL P395891